# EUROPEAN PATENT SPECIFICATION

(11) **EP 0 629 848 B1**
(45) Date of publication and mention of the grant of the patent: **11.11.1998**
(21) Application number: 94303751.5
(22) Date of filing: 25.05.1994
(51) Int. Cl.: G01N 3/56

(54) **Apparatus and method for determining the wear resistance of a fabric or polymeric material**
Vorrichtung und Verfahren zur Bestimmung der Verschleissfestigkeit eines Gewebes oder eines polymeren Werkstoffes
Dispositif et procédé pour déterminer la résistance à l'usure d'un tissu ou d'un matière polymère

(30) Priority: 27.05.1993 GB 9310981
(43) Date of publication of application: 21.12.1994
(73) Proprietor: JOHN HEATHCOAT & COMPANY LIMITED, Tiverton Devon EX16 5LL (GB)
(72) Inventor: Taylor, Charles Stephen, Tiverton, Devon EX16 4LL (GB); Buckley, Roy William, Halbarton, Devon (GB)
(74) Representative: Wilkinson, Stephen John

(56) References cited:
- DE-C- 831 336
- DE-C- 873 450
- GB-A- 1 092 172
- US-A- 1 749 297
- US-A- 2 788 655

## Description

The present invention relates to an apparatus for producing, in a test piece, a simulation of the effects of fatigue in a fabric or polymeric material. It further relates to a method of determining the effects of wear on a sample of fabric or polymeric material using the apparatus. The apparatus and the method described herein are particularly useful for determining the effects of surface abrasion and flexing in timing or transmission belts.

The effect of wear on a sample of a fabric or polymeric material is conventionally tested by placing the sample in contact with a flat stationary abrasive surface and then moving the sample across the abrasive surface while applying force to press the sample onto the abrasive surface. After a period of time the sample is removed and inspected for abrasion.

In this prior art method described above the emphasis is on surface abrasion and the extent of abrasion suffered by the sample is determined by visual inspection.

A realistic abrasion test would also, however, take the effect of flexing of the fabric or polymeric material into consideration. This is especially important if the results of testing are to be used to provide information on the extent of wear suffered by a fabric or polymeric material during normal usage which involves a strong element of flexing simultaneous with abrasion. For instance, in the normal use of transmission belts, such as belts used to drive industrial machinery or those used in automotives, friction and flexing are natural fatigue-imparting elements in the belt action. Such apparatus is known from DE-C-831 336.

The aim of the present invention is to provide an apparatus which imparts fatigue to a sample closer to that which is actually suffered by a fabric during ordinary use.

The present invention provides an apparatus for producing, in a test piece of a fabric or polymeric material, a simulation of the effects of fatigue suffered in normal use which apparatus comprises
- retaining means for retaining a test piece such that at least a portion thereof is maintained under predetermined tension;
- an abrading tool comprising a blade having a tip, preferably of a tungsten carbide rod, which in use contacts the surface of the portion of the test piece maintained under tension; and
- means for oscillating the blade through a predetermined amplitude such that in use the tip of the blade subjects the test piece to surface abrasion and flexing, characterized in that
the retaining means comprise a first clamp and a second clamp located away from the first clamp wherein the first and second clamps are adapted to retain opposite ends of a test piece and to maintain the test piece between the two clamps under constant tension.

Preferably, the blade of the abrading tool is pivotable about an axis such that when it oscillates the tip of the blade describes an arc. Obviously, if the tip of the blade at the mid position of the oscillation amplitude is set so that it presses into the fabric or polymeric material of the test piece, i.e., it deforms the test sample out of the plane in which the first and second clamps lie the test sample will be subjected to a substantial degree of flexing as well as friction in the region contacted by the tip of the blade during oscillation of the blade. It is, further, often useful to monitor the temperature build up in the abraded area during the test and, therefore, the apparatus may advantageously include a temperature probe, such as a thermocouple, for contacting the fabric or polymeric material during testing, which may be linked to a visual temperature indicator.

Any fabric, produced from natural or synthetic fibres or mixtures of these, and any polymeric material, such as a sheet or layer of an elastomeric material, e.g., rubbers, or plastics materials, e.g., thermoplastics, may be used as a test piece or sample in the apparatus of the present invention.

The apparatus of the invention is especially useful for studying the effects of wear on a test piece of a fabric or polymeric material to provide information about how the fabric or polymeric material suffers wear in normal use. In particular, we have found that the apparatus makes it possible to study the effects of fatigue which would be expected to be present in a transmission belt. To be able to do this we have found it desirable to use, as the test piece, a close simulation to the make-up and rigidity characteristics of a transmission belt so that conclusions about the effects of wear in the transmission belt during its normal use may be made on the basis of the information gained from the study of wear in the test piece using the apparatus. Thus, the invention further provides a method of studying the effects of wear in a transmission belt comprising
(1) taking a test piece of fabric-containing composite formed of a rigid backing member having on one surface thereof a layer of a vulcanized natural or synthetic rubber composition which, prior to being vulcanized is contacted with a layer of stretched fabric;
(2) locating the test piece in the apparatus of the invention with the fabric layer facing the abrading tool and retaining the test piece under predetermined tension by the retaining means;
(3) oscillating the blade of the abrading tool in contact with the fabric layer of the test piece for a period of time to subject the layer to friction and flexing;
(4) removing the test piece from the apparatus;
(5) peeling the rigid backing member from the test piece; and
(6) analysing the damage sustained by the fabric layer.

Typically, the damage sustained by the fabric layer in the test piece will be analysed by measuring the tensile strength of the fabric (or the threads/ filaments in the fabric). This makes it possible, by carrying out successive tests, to set up a numerical indication of the extent of wear imparted to a test piece. Considering that rubbing and flexing effects generate heat, the quantity of which depends on, for instance, the type of rubber composition used and the textile surface characteristics, it is often useful to monitor the temperature build up of the fabric during the test by incorporating a temperature probe, such as a thermocouple, directly between the fabric and the rubber layer, slightly embedded into the rubber. Such a probe may be linked to a digital readout.

For the construction of the test piece, the use of a rigid backing member in the composite makes it possible to fix the test piece in the apparatus under tension. We prefer to use a rigid backing member consisting of a nylon leno-weave fabric with the straight threads in the length direction to ensure maintenance of tension during testing. This also facilitates removal of the backing member from the test piece after subjecting the test piece to abrasion and flexing to allow analysis of the fabric to be carried out.

The choice of the natural or synthetic rubber composition used in the test piece will be determined by the type of composition used in the transmission belt being studied by virtue of the tests. Thus, the composition will, in addition to the rubber or polymeric component, also contain typical additives such as fillers, stabilizers, etc. The layer will have a substantially uniform and specified thickness since this will affect the flex heat generation and the degree of wear of the fabric in the test piece.

The fabric used will, obviously, be that used in the transmission belt being studied by virtue of the test. Typically, this will be pre-stretched prior to vulcanisation of the rubber composition to give a textile surface corresponding to that which in a transmission belt comes into contact with pulleys during use. We prefer to make the test piece according to the following method which simulates some of the conditions employed in the manufacture of a transmission belt. Firstly, fabric specimens are prepared. The fabric which has a construction, filament strength and denier identical or similar to those of a fabric used in transmission belt manufacture should be free of creases and distortion and should not be taken within 50 mm of the selvedge. The fabric to be tested is cut into two strips which are then sewn together at both ends to form a loop, and the fabric is then pre-stretched to approximately 50% linear extension by inserting a metal plate of the appropriate dimensions into the fabric loop. A composite is then formed by applying a layer of a natural or synthetic rubber composition in the 'green' state of a specified uniform thickness (typically about 3 mm) to one side of a rigid backing member (nylon leno-weave fabric wherein the straight threads of the leno weave run in the length direction of the composite strip). The pre-stretched loop of fabric is then applied to the exposed surface of the rubber composition and the composite strip is placed between hot plates in a press and the appropriate vulcanizing conditions of temperature, pressure and duration are applied to suit the particular rubber composition used. After cooling, the lose section of the textile fabric loop is discarded.

When the test piece is located on the apparatus it is the fabric layer that is contacted by the blade of the abrading tool. The test piece is preferably positioned such that the weft threads in the fabric run in the direction of the length of the piece (corresponding to the direction these threads have in a transmission belt), i.e., the direction of the vibration of the blade of the abrading tool. The test piece is fixed in the apparatus under a predetermined tension. This will usually be achieved by fixing one end of the test piece using the retaining means on the apparatus and then, prior to fixing the other end of the test piece, tensioning the test piece by using suspended weights so that this other end may be fixed while the test piece is under tension. The degree of tension employed will usually be determined having regard to the normal tension under which the transmission belt being studied by virtue of the test has during normal use. Useful results can, however, be obtained by applying a specific set tension to all test pieces. Our results have been achieved using a tension set at 1.4 kg.

For a better understanding of the present invention, some embodiments will now be described, by way of example, with reference to the accompanying drawings, in which:
Figure 1 is a side view of an apparatus according to the invention,
Figure 2a is a cross-section of an abrading tool used in the apparatus,
Figures 2b-2d are enlarged cross-sections of different abrading tools that may be used in the apparatus, and
Figure 3 is a view of another embodiment of the apparatus.

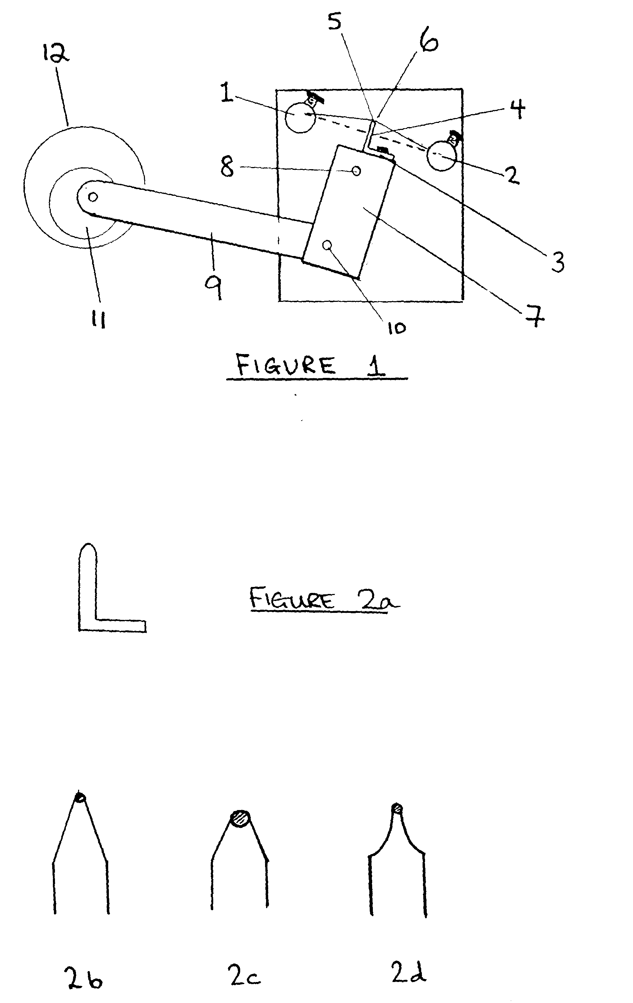

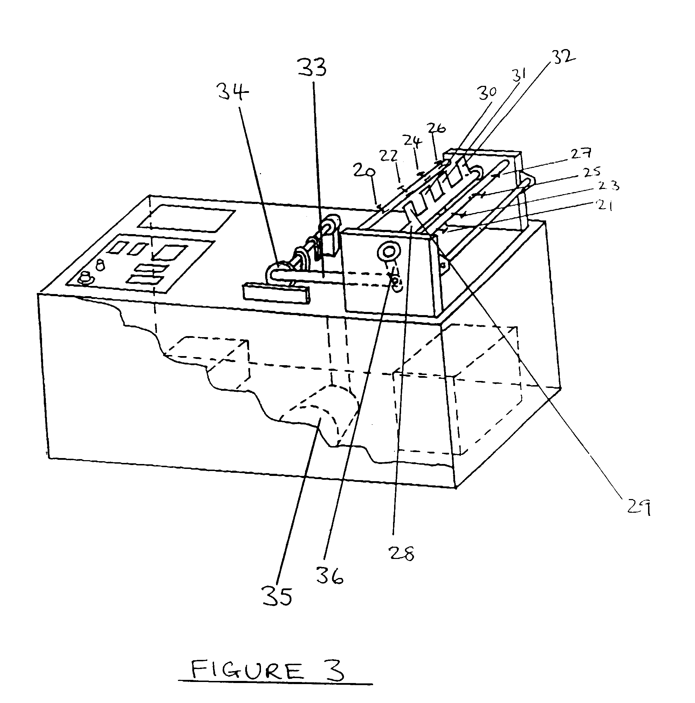

In Figure 1, the apparatus has retaining means (1, 2) for retaining opposite ends of a test piece or sample of a fabric or polymeric material. According to a preferred embodiment the sample used will be a fabric-containing composite constructed to simulate the characteristics of a transmission belt. An abrading tool 3 having a blade 4 with a tip 5 is located between the retaining means 1 and 2 such that when a test piece or sample of the fabric or polymeric material is fixed between the retaining means 1 and 2 the tip 5 of the blade 4 distorts the test piece or sample above the plane containing the retaining means at the point of contact 6. The tip 5 of the blade 4 of the abrading tool 3 has a cross-section shown in Figure 2a. According to a preferred embodiment the tip of the blade of abrasion tool is formed of tungsten carbide, typically in the form of a rod. A tool for a more severe abrasion effect with a tip consisting of a 1 mm diameter tungsten carbide rod is shown diagrammatically in Figure 2b. A tool tip for a less severe abrasion effect with a 3 mm diameter tungsten carbide rod is shown diagrammatically in Figure 2c. A shorter tool for use in abrasion resistance testing of the land area of a belt section is shown diagrammatically in Figure 2d, which makes it possible to avoid contact between the tool and the sides of the teeth of the belt section.

The tool 3 is set on block 7 which is pivotable about the pivot 8. The bottom of the block is pivotably attached to an arm 9 by a pivot 10 which in use is driven backwards and forwards by virtue of a cam 11 on motor 12 such that the block 7 is caused to pivot about the pivot 8. The pivoting of the block thus causes the edge of the blade to move to and fro describing an arc. It will be appreciated that the amplitude of the oscillation may be adjusted by adjusting the cam and the frequency of the oscillation may be adjusted by adjusting the speed of the motor. Thus, the amplitude and/or the frequency of the oscillation may be adjusted to simulate more closely the particular conditions under which a transmission belt operates. The temperature at the surface of the fabric may be monitored by a thermocouple (not shown). In the case where the test piece is a sample of a fabric-containing composite as herebefore described the thermocouple may conveniently be located between the fabric layer and the rubber layer. The thermocouple will typically be connected to a meter and/or to a pen recorder to provide a continuous read out. From the data obtained through the use of a thermocouple a graphical profile can be produced to show the rate of temperature increase and highest temperature reached during the test.

In Figure 3, a series of four sets of retaining means 20,21 22,23 24,25 and 26,27 are provided. A rotatable abrading tool 28 has four radial blades 29, 30, 31 and 32 and is caused to rotate by the movement of the arm 33 which is attached at one end to cam 34 driven by the motor 35 and which is pivotably attached at its other end to a radial member 36 extending from the tool 28. As the cam 34 revolves under the action of the motor the arm 33 moves the member 36 backwards and forwards thus causing the tool to rotate backwards and forwards and, thus, the blades to oscillate.

The apparatus shown in Figure 3 can be used to test up to four test pieces. It will be appreciated that the apparatus can be modified so that greater than four test pieces can be tested at any one time.

The apparatus may also be fitted with an environmental chamber so that the test pieces may be maintained, for the duration of the abrasion testing, under conditions, e.g., temperature and/or humidity, similar to those under which a transmission belt may be operated. The apparatus is preferably also fitted with digital readouts of, e.g., temperature at the surface of the test piece, oscillation frequency and amplitude and a timer.

The apparatus will be used to subject a test piece to surface abrasion and flexing for a suitable period of time which is long enough to produce damage in the test piece that can be analysed. We have found that a 3 hour period of testing gives sensible results in this respect. The test piece, after testing, is prepared for examination by removal of the rigid backing member. Following this, the fabric is typically subjected to tensile strength measurements so that the percentage loss of strength may be calculated and the filaments in the fabric may be subjected to examination, such as microscopic analysis, to provide useful information on the fundamental character of wear in the test piece.

## Claims

1. An apparatus for producing, in a test piece of a fabric or polymeric material, a simulation of the effects of fatigue suffered in normal use which apparatus comprises
- retaining means (1,2,20-27) for retaining a test piece under tension
- an abrading tool (3, 28) comprising a blade (4, 29-32) having a tip which in use contacts the surface of the portion of the test piece maintained under tension; and
- means (7-12) for oscillating the blade through a predetermined amplitude such that, in use, the blade subjects the test piece to surface abrasion and flexing, characterised in that the retaining means comprise a first clamp (1) and a second clamp (2) located away from the first clamp, wherein the first and second clamps are adapted to retain opposite ends of a test piece and to maintain the test piece between the two clamps under predetermined constant tension.

2. An apparatus according to claim 1, which also includes a temperature probe for contacting the test piece said probe being linked to a visual temperature indicator means.

3. An apparatus according to either claim 1 or claim 2, wherein the blade is pivotable about an axis such that when it oscillates through a predetermined amplitude the tip (5) of the blade describes an arc.

4. An apparatus according to any one of claim 1 to 3, having two or more test pieces.

5. An apparatus according to any one of claims 1 to 4, further comprising means for adjusting the amplitude of the oscillation.

6. An apparatus according to any one of claims 1 to 5, further comprising means for adjusting the frequency of the oscillation.

7. An apparatus according to any one of claims 1 to 6, wherein the abrading tool (3) is selected to have a blade (4) whose tip has a profile determined by the choice of fabric or polymeric material in the test piece.

8. An apparatus according to any one of claims 1 to 7, wherein the abrading tool (3) is positioned such that, during operation of the apparatus, the blade (4) of the tool at the mid-point of its oscillation presses into the fabric of the test piece.

9. An apparatus according to any one of claims 1 to 8, wherein the test piece is maintained in an environmental chamber.

10. A method of studying the effects of wear in a transmission belt comprising
1) taking a test piece of fabric-containing composite formed of a rigid backing member having on one surface thereof a layer of a vulcanized natural or synthetic rubber composition which, prior to being vulcanized, is contacted with a layer of stretched fabric;
2) locating said test piece in the apparatus of any one of claims 1 to 9 with the fabric layer facing the abrading tool (3, 28) and retaining the test piece under predetermined tension by the retaining means (1,2,20-27);
3) oscillating the blade (4, 29-32) of the abrading tool in contact with the fabric layer of the test piece for a period of time to subject the layer to friction and flexing;
4) removing the test piece from the apparatus;
5) peeling the rigid backing member from the test piece; and
6) analysing the damage sustained by the fabric layer.

11. A method according to claim 10, wherein the damage sustained by the fabric layer is analysed by measuring the tensile strength of the fabric layer.

## Patentansprüche

1. Vorrichtung zum Erzeugen, in einem Probestück eines Gewebe- oder Polymermaterials, einer Simulierung der im Normalgebrauch auftretenden Ermüdungserscheinungen, welche Vorrichtung umfasst
- Halteeinrichtungen (1, 2, 20 - 27), um ein Probestück unter Spannung zu halten
- ein Abnutzungsinstrument (3, 28), umfassend eine Klinge (4, 29 - 32) mit einer Schneide, die im Gebrauch die Oberfläche des unter Spannung gehaltenen Teils des Prüfkörpers berührt; und
- Einrichtungen (7 - 12), um die Klinge über eine vorbestimmte Amplitude hin- und herzubewegen, so dass die Klinge im Gebrauch das Probestück einer Oberflächenabnutzung und einer Biegung unterwirft, dadurch gekennzeichnet, dass die Halteeinrichtungen eine erste Klemmvorrichtung (1) und eine entfernt von der ersten Klemmvorrichtung angeordnete zweite Klemmvorrichtung (2) umfassen, wobei die erste und zweite Klemmvorrichtung angepasst sind, um entgegengesetzte Enden eines Probestücks festzuhalten und das Probestück zwischen den beiden Klemmvorrichtungen unter vorbestimmter konstanter Spannung zu halten.

2. Vorrichtung nach Anspruch 1, welche auch eine Temperatursonde zum Berühren des Probestücks einschließt, wobei die Sonde mit einer optischen Temperaturanzeige-Einrichtung verbunden ist.

3. Vorrichtung nach Anspruch 1 oder Anspruch 2, bei welcher die Klinge um eine Achse schwenkbar ist, so dass, wenn sie sich über eine vorbestimmte Amplitude hin- und herbewegt, die Schneide (5) der Klinge einen Bogen beschreibt.

4. Vorrichtung nach einem beliebigen der Ansprüche 1 bis 3, mit zwei oder mehr Probestücken.

5. Vorrichtung nach einem beliebigen der Ansprüche 1 bis 4, weiter umfassend Einrichtungen zum Einstellen der Amplitude der Hin- und Herbewegung.

6. Vorrichtung nach einem beliebigen der Ansprüche 1 bis 5, weiter umfassend Einrichtungen zum Einstellen der Frequenz der Hin- und Herbewegung.

7. Vorrichtung nach einem beliebigen der Ansprüche 1 bis 6, bei welcher das Abnutzungsinstrument (3) so ausgewählt ist, dass es eine Klinge (4) aufweist, deren Schneide ein Profil besitzt, das durch die Wahl des Gewebe- oder Polymermaterials im Probestück bestimmt ist.

8. Vorrichtung nach einem beliebigen der Ansprüche 1 bis 7, bei welcher das Abnutzungsinstrument (3) so angeordnet ist, dass während des Betriebs der Vorrichtung die Klinge (4) des Instruments in der Mitte seiner Hin- und Herbewegung in das Gewebe des Probestücks drückt.

9. Vorrichtung nach einem beliebigen der Ansprüche 1 bis 8, bei welcher das Probestück in einer Klimakammer gehalten wird.

10. Verfahren zum Untersuchen der Verschleißerscheinungen in einem Treibriemen, umfassend
1) Nehmen eines Probestücks von gewebehaltigem Verbundmaterial, das von einem steifen Trägerelement gebildet wird, welches auf seiner einen Oberfläche eine Schicht einer vulkanisierten Natur- oder Synthese-Kautschukzusammensetzung aufweist, die vor ihrer Vulkanisation mit einer Schicht gedehntem Gewebe in Berührung gebracht wird;
2) Anordnen des besagten Probestücks in der Vorrichtung gemäß einem beliebigen der Ansprüche 1 bis 9, wobei die Gewebeschicht dem Abnutzungsinstrument (3, 28) gegenüberliegt, und Halten des Probestücks unter vorbestimmter Spannung mittels der Halteeinrichtungen (1, 2, 20 - 27);
3) Hin- und Herbewegen der Klinge (4, 29 - 32) des Abnutzungsinstruments in Berührung mit der Gewebeschicht des Probestücks über eine Zeitspanne, um die Schicht einer Reibung und Biegung zu unterwerfen;
4) Entfernen des Probestücks aus der Vorrichtung;
5) Abschälen des steifen Trägerelements vom Probestück; und
6) Analysieren des Schadens, den die Gewebeschicht erlitten hat.

11. Verfahren nach Anspruch 10, bei dem der Schaden, den die Gewebeschicht erlitten hat, durch Messung der Zugfestigkeit der Gewebeschicht analysiert wird.

## Revendications

1. Appareil de production, dans une éprouvette d'une étoffe ou d'un matériau polymère, d'une simulation des effets de la fatigue subie pendant l'utilisation normale, l'appareil comprenant :
- un dispositif (1, 2, 20-27) de retenue d'une éprouvette sous tension,
- un outil (3, 28) d'abrasion qui comporte une lame (4, 29-32) ayant un bout qui, pendant l'utilisation, est au contact de la surface de la partie de l'éprouvette maintenue sous tension, et
- un dispositif (7-12) destiné à faire osciller la lame avec une amplitude prédéterminée telle que, pendant l'utilisation, la lame fait subir à l'éprouvette une abrasion de sa surface et une flexion, caractérisé en ce que le dispositif de retenue comporte une première pince (1) et une seconde pince (2) placée à distance de la première pince, et la première et la seconde pince sont destinées à retenir les extrémités opposées d'une éprouvette et à maintenir l'éprouvette entre les deux pinces sous une tension constante prédéterminée.

2. Appareil selon la revendication 1, qui comporte en outre une sonde de température destinée à être au contact de l'éprouvette, la sonde étant reliée à un dispositif indicateur visuel de température.

3. Appareil selon la revendication 1 ou 2, dans lequel la lame peut pivoter autour d'un axe afin que, lorsqu'elle oscille avec une amplitude prédéterminée, le bout (5) de la lame décrive un arc.

4. Appareil selon l'une quelconque des revendications 1 à 3, possédant au moins deux éprouvettes.

5. Appareil selon l'une quelconque des revendications 1 à 4, comprenant en outre un dispositif d'ajustement de l'amplitude d'oscillation.

6. Appareil selon l'une quelconque des revendications 1 à 5, comprenant en outre un dispositif d'ajustement de la fréquence de l'oscillation.

7. Appareil selon l'une quelconque des revendications 1 à 6, dans lequel l'outil d'abrasion (3) est sélectionné afin qu'il possède une lame (4) dont le bout a un profil déterminé par la sélection de l'étoffe ou du matériau polymère de l'éprouvette.

8. Appareil selon l'une quelconque des revendications 1 à 7, dans lequel l'outil d'abrasion (3) est disposé de manière que, pendant le fonctionnement de l'appareil, la lame (4) de l'outil, au point médian de son oscillation, exerce une pression sur l'étoffe de l'éprouvette.

9. Appareil selon l'une quelconque des revendications 1 à 8, dans lequel l'éprouvette est maintenue dans une chambre de création d'une atmosphère.

10. Procédé d'étude des effets de l'usure sur une courroie de transmission, comprenant :
1) le prélèvement d'une éprouvette d'un matériau composite contenant une étoffe, formé d'un organe rigide de renforcement, ayant sur une surface une couche d'une composition de caoutchouc naturel ou synthétique vulcanisé qui, avant vulcanisation, est mise au contact d'une couche d'étoffe tendue,
2) le positionnement de l'éprouvette dans l'appareil selon l'une quelconque des revendications 1 à 9, la couche d'étoffe étant tournée vers l'outil d'abrasion (3, 28) et retenant l'éprouvette sous une tension prédéterminée à l'aide du dispositif de retenue (1, 2, 20-27),
3) l'oscillation de la lame (4, 29-32) de l'outil d'abrasion au contact de la couche d'étoffe de l'éprouvette pendant une période telle que la couche est soumise à un frottement et une flexion,
4) l'enlèvement de l'éprouvette de l'appareil,
5) la séparation par pelage de l'organe rigide d'appui de l'éprouvette, et
6) l'analyse de la détérioration subie par la couche d'étoffe.

11. Procédé selon la revendication 10, dans lequel la détérioration subie par la couche d'étoffe est analysée par mesure de la résistance à la traction de la couche d'étoffe.
